# EUROPEAN PATENT SPECIFICATION

(11) **EP 0 428 723 B1**
(45) Date of publication and mention of the grant of the patent: **31.01.1996**
(21) Application number: 88906129.7
(22) Date of filing: 26.07.1988
(51) Int. Cl.: A61B 5/14

(54) **BLOOD COLLECTING DEVICE**
BLUTSAMMELANORDNUNG
DISPOSITIF POUR PRELEVEMENTS SANGUINS

(43) Date of publication of application: 29.05.1991
(73) Proprietor: TERUMO KABUSHIKI KAISHA, Tokyo 151 (JP)
(72) Inventor: KASAI, Masaaki, Nakakoma-gun Yamanashi-ken 409-38 (JP)
(74) Representative: Casalonga, Axel
(86) International application number: JP8800745
(87) International publication number: WO9000880

(56) References cited:
- WO-A-82/04387
- FR-A- 1 384 386
- JP-A- 6 190 643
- JP-A-60 232 134
- JP-B- 4 739 997
- NL-A- 7 005 292
- US-A- 4 295 476

## Description

This invention relates to a blood collector for use in collecting blood.

A variety of blood collectors have been used in clinical and biochemical examinations, for example. Most commonly used blood collectors are of the type comprising a blood collecting tube whose interior is kept under reduced pressure and a holder capable of receiving the blood collecting tube therein and having a puncture needle at a front end. The blood collecting tube includes a tubular member having an open end and a plug closing the open end. The tubular member is formed of glass, synthetic resins or similar materials and the plug is generally formed of rubber materials such as vulcanized butyl rubber. The interior of the blood collecting tube is kept under reduced pressure. The holder is a cylindrical member having a rear end through which the blood collecting tube is inserted into the cylindrical member and a front end at which a puncture needle is mounted. The puncture needle includes a blood collecting needle segment extending in a forward direction and adapted to be punctured into a human body or the like and a hollow needle segment extending in a rearward direction, and further includes a hub disposed intermediate the blood collecting needle segment and the hollow needle segment, through which the puncture needle is mounted in the holder. The puncture needle is provided with a resilient sheath enclosing the hollow needle segment, for example, a rubber cap.

The blood collector of the above-mentioned construction is used to collect blood. First, the blood collecting needle segment which is the front end of the puncture needle mounted in the holder is thrusted into a blood vessel. Then the blood collecting tube is inserted into the holder until the free end of the hollow needle segment which is the rear end of the puncture needle punctures throughout the plug of the blood collecting tube and reaches the interior space of the blood collecting tube. At this point, the rubber cap is folded in a bellows shape between the plug and the holder.

There is the risk that the blood collecting tube can be moved back and separated from the needle or "kicked back" by the reaction of the rubber cap if the friction between the plug and the hollow needle segment is low. Choice must be made of a plug capable of preventing kick-back. The use of such a plug creates the problem that the hollow needle segment cannot be readily thrusted into the plug because the plug has an increased puncture resistance.

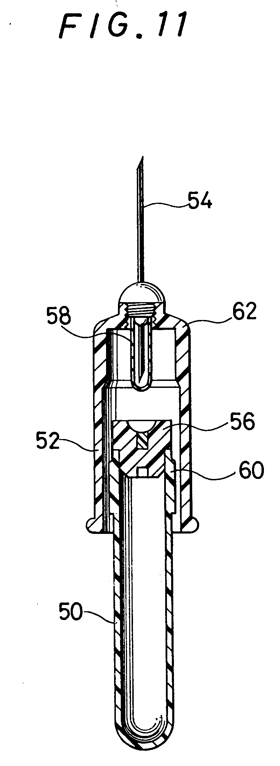

To solve the problem, the applicant proposed a blood collector as disclosed in Japanese Patent Application Kokai No. 61-90643. This blood collector is shown in FIG. 11. In the blood collector shown in FIG. 11, an open end of a tubular member 50 is closed with a plug 56 to form a blood collecting tube and a holder 52 has a puncture needle 54 at a front end. A rear end portion of the puncture needle 54 is enclosed in a rubber cap 58. The tubular member 50 is provided on the outer surface of its front portion with two axially extending ribs 60. The inside diameter of the front portion of the holder 52 is reduced to form a constricted portion 62 in correspondence with the ribs. The ribs 60 engage the constricted portion 62 of the holder to prevent the rubber cap from kicking back the blood collecting tube.

As another solution to the above-mentioned problem, the applicant proposed a blood collector as disclosed in Japanese Patent Application Kokai No. 60-232134. In this blood collector, a rubber plug which forms a closure for a blood collecting tube is provided on the outer surface with axially extending ribs such that the ribs are in contact with the inside surface of a holder. Kick-back is prevented by the friction between the ribs on the plug and the holder.

As a further solution to the above-mentioned problem, the applicant proposed a blood collector as disclosed in Japanese Patent Application Kokai No. 61-90644. In this blood collector, a holder for a blood collecting tube is provided on the inside surface with protruding ribs. Kick-back is prevented by the engagement between the blood collecting tube itself or a plug therefor and the ribs on the holder. As compared with the prior art blood collectors of the type in which kick-back is prevented by the friction between a rubber plug and a hollow needle segment, these blood collectors are advantageous in that the blood collecting tube can be readily inserted into the holder. However, since kick-back is prevented by the frictional force due to the contact between the blood collecting tube itself or a plug therefor and the inside surface of the holder, a substantial force is necessary to insert the blood collecting tube into the holder. Additionally, a substantial force is also necessary when the blood collecting tube is withdrawn from the holder.

US-A-4 295 476 discloses a blood collector comprising a blood collecting tube, a puncture needle, a resilient sheath enclosing part of the puncture needle and a holder for holding the blood collecting tube. This blood collector is provided with a spring-depressible tongue which has to be permanently depressed by the finger of the user in order to retain the collecting tube. After releasing the depressing force, the tongue is adapted to spring back to be substantially flush with the side wall.

Further, WO-A-204 387 discloses snapping means for preventing relative movement of the tube and the holder. The tube has a close fit with the holder.

An object of the present invention is to provide a blood collector comprising a blood collecting tube and holder wherein the blood collecting tube can be readily moved into and out of the holder while preventing the blood collecting tube from being kicked back.

As claimed, a blood collector comprises a blood collecting tube having an open end which is closed with a closure member; a puncture needle including a needle tube having a blood collecting needle segment extending in one direction and a hollow needle segment extending in another direction, and a resilient sheath enclosing the hollow needle segment. A holder is provided for holding the blood collecting tube, in the form of a hollow member having an open rear end, the blood collecting tube being removably inserted into the holder through the rear end, the holder being designed such that the blood collecting tube may be inserted into the holder without bringing the outside surface into substantial contact with the inside surface of the holder. Further, the puncture needle is mounted at the front end of the hollow member such that the blood collecting needle segment of the puncture needle extends beyond the front end of the hollow member and the hollow needle segment extends through the hollow member. The holder and the blood collecting tube are further provided with a pair of retaining means which engage with each other when the blood collecting tube is inserted into the holder.

According to the invention the inner diameter of the holder and the outer diameter of the blood collecting tube are provided such that there is a clearance between the holder and the blood collecting tube inserted therein to allow the blood collecting tube to be slanted and/or rotated within the holder to thereby achieve engagement therebetween, which engagement provides a retaining force greater than the reaction of the resilient sheath which is axially compressed by the closure member of the blood collecting tube and ensuring safe engagement of the holder and the tube.

In the following advantageous embodiments of the invention are described for further elucidating the invention. In the drawings:
FIG. 1 is a cross section showing a blood collector according to one embodiment of the present invention.
FIG. 2 is a cross section of the holder for the blood collecting tube of FIG. 1, taken along retaining means.
FIG. 3 is a cross section showing a blood collector according to another embodiment of the present invention.
FIG. 4a is a cross section of the holder for the blood collecting tube of FIG. 3, taken along retaining means.
FIG. 4b is a rear view of the blood collecting tube of FIG. 3.
FIG. 5 is a cross section showing a blood collector according to a further embodiment of the present invention.
FIG. 6 is a cross section taken along lines X-X of the holder for the blood collecting tube of FIG. 5.
FIG. 7 is a cross section showing a blood collector according to a still further embodiment of the present invention.
FIG. 8 is a cross section showing a blood collector according to a yet further embodiment of the present invention.
FIG. 9 is a cross section of the holder for the blood collecting tube of FIG. 8, taken along retaining means.
FIG. 10 is a rear view of the blood collecting tube of FIG. 8.
FIG. 11 is a cross-sectional view showing a prior art blood collector.

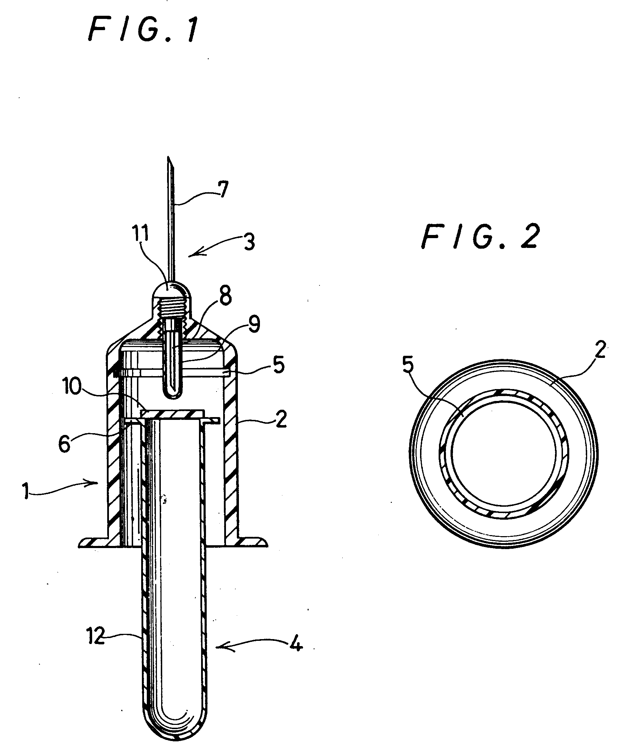

The blood collector of the present invention is described by referring to one embodiment shown in FIG. 1.

The blood collector 1 of the present invention is illustrated as comprising a blood collecting tube 4 having an open end which is closed with a closure member 10, a puncture needle 3 including a needle tube having a blood collecting needle segment 7 extending in one direction and a hollow needle segment 8 extending in another direction, and a resilient sheath 9 enclosing the hollow needle segment 8, and a holder 2 for holding the blood collecting tube 4, in the form of a hollow member having an open rear end, the blood collecting tube 4 being removably inserted into the holder 2 through the rear end, and the puncture needle 3 being mounted at the front end of the hollow member such that the blood collecting needle segment 7 of the puncture needle 3 extends beyond the front end of the hollow member and the hollow needle segment 8 extends through the hollow member. The holder 2 is designed such that the blood collecting tube 4 may be inserted into the holder 2 without substantial contact with the inside surface of the holder 2. Further, the holder 2 and the blood collecting tube 4 are provided with a pair of retaining means 5 and 6. The retaining means 5 and 6 engage with each other when the blood collecting tube 4 is inserted into the holder 2 until the hollow needle segment 8 punctures the closure member 10, to thereby provide a retaining force greater than the reaction of the resilient sheath 9 which is axially compressed by the closure member 10 of the blood collecting tube 4.

More illustratively, the blood collector 1 of the present invention includes the blood collecting tube 4, the holder 2 into which the blood collecting tube 4 can be inserted, and the puncture needle 3 mounted at the front end of the holder 2.

The blood collecting tube 4 includes a closed bottom tubular member 12 having one open end and a closure member 10 air tightly closing the opening of the tubular member 12. The interior of the blood collecting tube 4 is kept under reduced pressure. The material of which the tubular member 12 is made may have high gas-barrier properties and is preferably selected from glass, polyvinyl chloride, polyethylene terephthalate and the like. In case the tubular member 12 is formed from a poor gas-barrier material, the surface (for example, outer surface) of the tubular member 12 is preferably coated with a layer having gas barrier properties such as polyvinyl alcohol and ethylene-vinyl alcohol copolymers. When the tubular member 12 is formed of a polyvinyl chloride resin, the inside surface of the tubular member 12 is preferably coated with an anti-dissolving agent in order to prevent a stabilizer contained in the polyvinyl chloride resin from being dissolved out in blood being collected.

Depending on the intended application, an anticoagulant agent such as heparin powder and EDTA-2K may be applied to the inside surface of the tubular member 12 or contained therein. Conversely, a coagulation promoter may be applied to the inside surface of the tubular member 12 or contained therein. A serum separating agent may also be contained in the tubular member 12.

The tubular member 12 on the outer surface is provided with an outwardly extending annular rib which forms the retaining means 6 of the blood collecting tube 4. The retaining means 6 engages the retaining means 5 of the holder 2 to be described later, thereby preventing removal of the blood collecting tube 4 from the holder 2. The retaining means 6 of the blood collecting tube 4 may be of any desired shape insofar as it can engage with the retaining means 5 of the holder 2. For example, the tubular member 12 may be provided with a plurality of outwardly extending geartooth-like ribs, one or more semi-circular or crescent ribs, or one or more axially extending short ribs. Preferred retaining means is an annular rib extending perpendicular to the axis of the tubular member 12 as shown in FIG. 1. The annular rib facilitates engagement with the retaining means of the holder because the annular rib is retaining means provided over the entire periphery of the tubular member 12. That surface of the retaining means 6 of the blood collecting tube 4 which faces to the rear side of the tubular member 12 is preferably made flat. The retaining means having a flat rear surface ensures that the retaining means contacts and engages the retaining means 5 of the holder 2. The rib or retaining means 6 of the blood collecting tube 4 is dimensioned such that it protrudes a distance of from 0.5 mm to 5.0 mm, preferably from 1.0 mm to 2.5 mm beyond the outside surface of the blood collecting tube 4.

The closure member 10 attached to the opening of the tubular member 12 in a gas-tight manner is a member into which the hollow needle segment 8 of the puncture needle 3 mounted to the holder 2 can be stuck. The closure member 10 has such a strength that it is not broken by the reaction of the resilient sheath 9 surrounding the hollow needle segment 8. Since the blood collector 1 of the present invention should allow the blood collecting tube 4 be slanted and/or rotated after the hollow needle segment 8 has been thrusted into the closure member 10, the friction between the closure member 10 and the hollow needle segment 8 is of a sufficient order to allow such slanting or rotation. The closure member 10 meeting the above-mentioned requirements is preferably a laminate comprising a lower gas-barrier member having a high gas-tightness, an upper sealing member bonded to the lower gas-barrier member, and an adhesive film bonded to the lower surface of the gas-barrier member. The closure member 10 is mounted to the tubular member 12 by attaching its adhesive film to the edge of the opening of the tubular member 12 in a gas-tight manner. The relationship of the gas-barrier member and the sealing member may be reversed. Instead of the layer, the sealing member may be formed by bonding a sealing material to the surface (or rear surface) of a central portion of the gas-barrier member. The gas-barrier member must be of a material capable of maintaining the interior of the tubular member 12 under reduced pressure. The gas-barrier member may be formed of, for example, metal foils such as aluminum foils, and resin films such as ethylenevinyl alcohol copolymers and polyvinylidene chloride. The sealing member must be of a material capable of sealing a puncture opening to maintain liquid tightness both when the hollow needle segment 8 is thrusted into and withdrawn from the closure member 10. The sealing member may be formed of, for example, rubber materials such as natural rubber, isoprene rubber, styrene-butadiene rubber and silicone rubber, and resins such as thermoplastic elastomers.

Although the closure member of film shape is referred to in the foregoing description, the present invention is not limited thereto. For example, a deformed plug may be used which is prepared by starting with a plug or rubber plug of a prior art shape (as shown in FIG. 11), reducing the thickness of a central portion thereof to reduce the friction with the hollow needle segment, and covering at least the surface (or rear surface) of the plug near the reduced thickness portion, more reliably all the surface portion which is exposed outside when the plug is mounted to the tubular member, with a gas-barrier member.

Although the retaining means 6 is provided by forming a rib on the tubular member 12 as the blood collecting tube 4 in the illustrated embodiment, the present invention is not limited thereto. The closure member 10 may be used to form the retaining means. For example, a member having greater dimensions than the opening of the tubular member 12 is used as the closure member 10 so that the portion of the closure member that extends beyond the opening of the tubular member 12 forms the retaining means. In this case, the outer peripheral portion of the closure member must be hard enough to engage the retaining means of the holder. If the outer peripheral portion of the closure member is not hard enough, the retaining means capable of engaging the retaining means of the holder may be accomplished by applying an annular reinforcement to the surface or rear surface of the closure member. The shape of the rib or reinforcement used herein is similar to the embodiment in which the tubular member 12 is provided with a rib.

Although a rib is used as the retaining means of the blood collecting tube in the above-illustrated embodiment, the present invention is not limited thereto. Conversely, the tubular member forming the blood collecting tube may be provided on the outer surface with an annular, semi-circular or crescent groove to form retaining means. In this case, that surface of the groove which is located on the front end of the blood collecting tube 4 engages the retaining means of the holder.

The holder 2 for the blood collecting tube 4 is a hollow member having an open rear end and a front end where a thread (internal thread) is formed to receive the puncture needle 3 therein. The puncture needle 3 has the hub 11 where a thread (external thread) is formed. The puncture needle 3 is mounted to the holder 2 by screwing the threaded hub into the threaded bore at the front end of the holder. Otherwise, the hub of the puncture needle may be directly secured to the holder.

The puncture needle 3 includes the blood collecting needle segment 7 extending in one direction to be stuck into the blood vessel, the hollow needle segment 8 extending in the other direction (opposite to the blood collecting needle segment 7) to be stuck into the closure member 10 of the blood collecting tube 4 when the blood collecting tube 4 is inserted into the holder 2, and the hub 11. The puncture needle 3 further includes the resilient sheath 9 enclosing the hollow needle segment 8. The resilient sheath is generally formed of rubbery material.

The shape of the holder 2 must be such that the blood collecting tube 4 may be readily inserted therein. To this end, in the blood collector of the present invention, the holder 2 is designed such that the blood collecting tube 4 may be inserted into the holder 2 without bringing the outside surface of the tube into substantial contact with the inside surface of the holder. The term "without substantial contact" means that there is a sufficient clearance between the holder 2 and the blood collecting tube 4 inserted therein to allow the blood collecting tube 4 to be slanted and/or rotated within the holder 2. Then the blood collecting tube 4 can be inserted into the holder 2 without substantial resistance. The holder 2 is provided on the inside surface with the retaining means 5. In the embodiment shown in FIG. 1, the retaining means 5 of the holder 2 is illustrated as an annular recess extending perpendicular to the axis of the holder 2. FIG. 2 is a cross section of the holder 2 taken along the retaining means 5 of the holder 2. The retaining means 5 provided on the holder 2 engages the retaining means 6 of the blood collecting tube 4 to prevent the blood collecting tube 4 from being moved away from the holder 2 by the reaction of the resilient sheath 9 of the puncture needle 3. That is, the engagement between the retaining means 6 of the blood collecting tube 4 and the retaining means 5 of the holder 2 provides a retaining force greater than the reaction of the resilient sheath 9 which is axially compressed by the closure member 10 when the hollow needle segment 8 of the puncture needle 3 is thrusted into the closure member 10 of the blood collecting tube 4. In the blood collector 1 of the embodiment shown in FIG. 1, when the blood collecting tube 4 is inserted into the holder 2 until the hollow needle segment 8 punctures the closure member 10 of the blood collecting tube 4, the rib or retaining means 6 of the blood collecting tube 4 partially enters the groove or retaining means 5 of the holder 2 to achieve engagement therebetween. At this point, the blood collecting tube 4 extends at a slight angle with respect to the axis of the holder 2. If the retaining means 6 of the blood collecting tube 4 does not spontaneously engage the retaining means of the holder 2, the blood collecting tube 4 is slightly slanted with respect to the axis of the holder 2 to thereby cause the rib or retaining means 6 of the blood collecting tube 4 to partially enter the groove or retaining means 5 of the holder 2 to achieve engagement therebetween. The engagement of part of the retaining means of the blood collecting tube with part of the retaining means of the holder provides a retaining force greater than the reaction of the resilient sheath 9, preventing the blood collecting tube 4 from being moved away from the holder 2.

The retaining means 5 of the holder 2 may be of any desired shape insofar as it can engage the retaining means 6 of the blood collecting tube 4. For example, where the retaining means of the blood collecting tube is geartooth-like ribs, the retaining means of the holder may be a plurality of recesses having a geartooth-like cross section engageable with the ribs or retaining means of the blood collecting tube. Where the retaining means of the blood collecting tube is an annular, semi-circular or crescent rib(s), the holder may be provided with one or more semi-circular or crescent recesses engageable with the rib(s) as its retaining means. Where the retaining means of the blood collecting tube is an axially extending rib(s), the holder may be provided with one or more axially extending recesses engageable with the rib(s) as its retaining means. The preferred retaining means 5 of the holder 2 is an annular recess in a plane perpendicular to the axis and the preferred retaining means 6 of the blood collecting tube 4 is an annular rib as shown in FIG. 1. With this combination, the retaining means 5 and 6 can be engaged with each other readily and reliably because the retaining means 5 of the holder 2 extends over the entire inside circumference of the holder 2 and the retaining means 6 of the blood collecting tube 4 extends over the entire outside circumference of the blood collecting tube 4. That surface of the retaining means 5 of the holder 2 which is located on the rear side of the holder 2 is preferably made flat. The retaining means having a flat rear surface ensures that the retaining means contacts and engages the rib or retaining means 6 of the blood collecting tube 4. The recess or retaining means 5 of the holder 2 has a depth of from 0.2 mm to 2.0 mm, preferably from 0.5 mm to 1.0 mm.

If neither of the retaining means 6 and 5 of the blood collecting tube 4 and the holder 2 is a continuous annular one, the blood collecting tube 4 is inserted into the holder 2 and then rotated until the retaining means 6 of the blood collecting tube 4 engages the retaining means 5 of the holder 2.

The retaining means 5 of the holder 2 and the retaining means 6 of the blood collecting tube 4 are located such that they may engage with each other when the hollow needle segment 8 has punctured throughout the closure member 10 of the blood collecting tube 4. Therefore, the retaining means 5 and 6 need not be located at the front end of the blood collecting tube 4 and the holder 2. They may be spaced a predetermined distance from the front end of the blood collecting tube 4 and the holder 2.

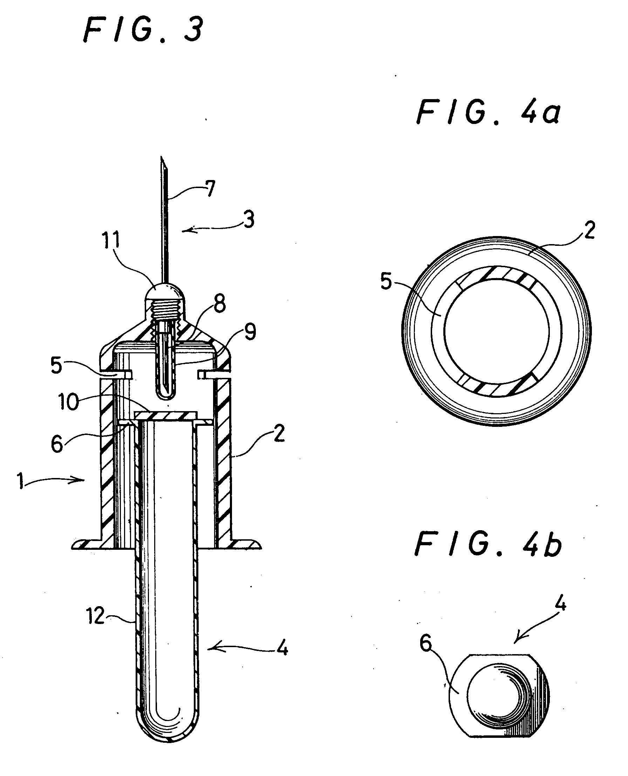

Next, the blood collector according to another embodiment of the present invention is described with reference to FIG. 3. Like parts are designated by like numerals as in FIG. 1.

The difference between the blood collector 1 of this embodiment and the blood collector of FIG. 1 is the configuration of the retaining means 5 provided in the inside surface of the holder 2 for the blood collecting tube. In this blood collector 1, the retaining means 5 of the holder 2 is formed by two slots which communicate the interior and the exterior of the holder 2. FIG. 4a is a cross section of the holder 2 taken along the slots. The retaining means 6 of the blood collecting tube 4 enters the slots to accomplish the engagement therebetween. The slots have a sufficient width to receive the retaining means 6 of the blood collecting tube 4. The longer the slots, the easier is their engagement with the retaining means of the blood collecting tube. However, too long slots will reduce the strength of the holder 2. Thus the length of the slots ranges from 2/10 to 7/10, preferably from 4/10 to 6/10 of the entire circumference of the holder 2. A plurality of slots are preferably formed as shown in FIG. 4a. Most preferably two slots are formed in an opposed relationship. In this case, the length of each slot ranges from 1/10 to 3.5/10, preferably from 2/10 to 3/10 of the entire circumference of the holder 2. The retaining means 6 of the blood collecting tube 4 is preferably one capable of reliable engagement with the retaining means 5 of the holder 2. To this end, the retaining means 6 of the blood collecting tube 4 is preferably of a length matching with the shape of the retaining means 5 of the holder 2 (that is, a length shorter than the slot in the holder) as shown in FIG. 4b.

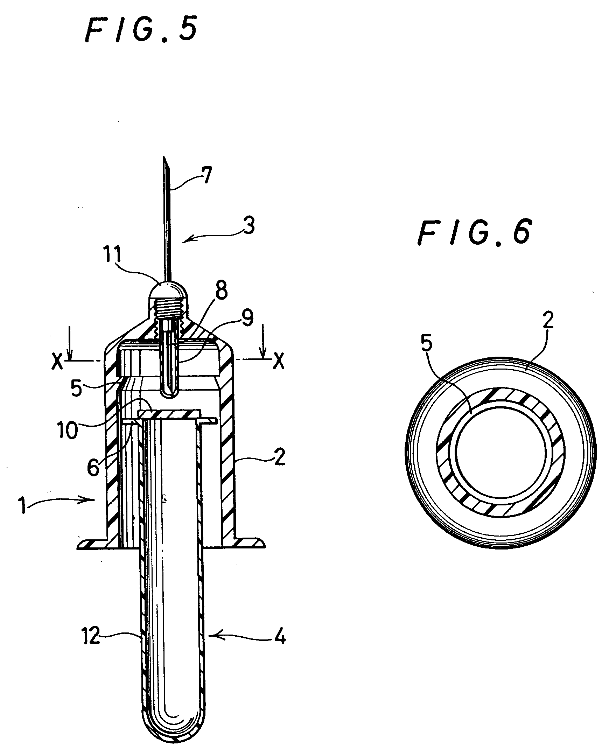

Next, the blood collector according to a further embodiment of the present invention is described with reference to FIG. 5. Like parts are designated by like numerals as in FIG. 1.

The blood collecting tube 4 of the blood collector 1 of this embodiment is the same as the blood collecting tube of FIG. 1. The difference from the blood collector of FIG. 1 is the configuration of the retaining means 5 provided in the inside surface of the holder 2 for the blood collecting tube. In FIG. 5, the retaining means 5 of the holder 2 is formed by an annular rim extending inwardly from the holder 2. FIG. 6 is a cross section of the holder taken along lines X-X in FIG. 5. The holder 2 is dimensioned such that the blood collecting tube 4 may be readily inserted therein, ensuring that the blood collecting tube 4 can be inserted into the holder 2 without substantial contact between the outside surface of the blood collecting tube 4 and the inside surface of the holder 2. Even at the location of the annular rim, the blood collecting tube 4 can be inserted into the holder 2 without substantial contact between the outside surface of the blood collecting tube 4 including the retaining means 6 and the inside surface of the holder 2. More particularly, the crest of the annular rim provided on the holder 2 has an inside diameter which is larger than the outer diameter of the retaining means 6 of the blood collecting tube 4. The annular rim need not be necessary annular while it is contemplated to use a series of serrations to form a gear shape, or one or more semi-circular or crescent rims. Preferred is an annular rim extending perpendicular to the axis of the holder 2 as shown in FIG. 5. The annular rim ensures reliable engagement with the retaining means 6 of the blood collecting tube 4 because the retaining means 5 of the holder 2 extends over the entire inside circumference of the holder 2. That surface of the annular rim which is located on the front side of the holder 2 is preferably made flat. The annular rim having a flat front surface ensures contact and engagement with the rib or retaining means 6 of the blood collecting tube 4. The annular rim is dimensioned such that the width of the rim extending from the inside surface of the holder 2 ranges from 0.2 mm to 2.0 mm, preferably from 0.5 mm to 1.0 mm. Further, that surface of the annular rim which is located on the rear side of the holder is preferably made a moderate slant surface as shown in FIG. 5. With the slant surface, the blood collecting tube 4 can be readily inserted into the holder 2 without the rib or retaining means 6 of the blood collecting tube 4 being hooked by the annular rim. Conversely, that surface of the retaining means 6 of the blood collecting tube 4 which is located on the front side of the blood collecting tube may be made a moderate slant surface as above.

In the blood collector 1 having the blood collecting tube 4 and the holder 2 provided with retaining means 6 and 5 as shown in FIG. 5, when the blood collecting tube 4 is inserted into the holder 2, the retaining means 6 of the blood collecting tube 4 spontaneously engages the retaining means of the holder 2 at the time when the former has passed the latter. The blood collecting tube is at a slight angle with respect to the holder at this point. If the retaining means do not engage with each other spontaneously, they can be readily engaged by slightly slanting the blood collecting tube. The engagement of the retaining means 5 and 6 prevents the blood collecting tube 4 from being moved away from the holder 2.

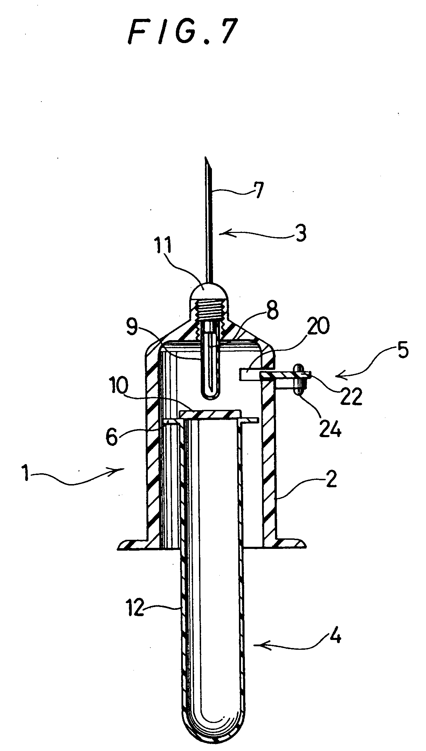

Although the rim secured to the inside surface of the holder is described in the illustrated embodiment, the present invention is not limited thereto. For example, as shown in FIG. 7, the holder 2 is provided with a slot 20 which communicates the interior and the exterior of the holder 2. A plate or rod-shaped member 22 is inserted into the slot 20 to form the retaining means of the holder 2 which is to engage the retaining means 6 of the blood collecting tube 4. Preferably, the member 22 is rotatably connected to the outside surface of the holder 2 through a fixture 24 as shown in FIG. 7. The pivotal connection facilitates movement of the member 22 into and out of the slot 20. The shape of the lower surface of the member 22 is preferably the same as or similar to the outside shape of the blood collecting tube. This ensures engagement of the member 22 with the retaining means 6 of the blood collecting tube 4. The embodiment shown in FIG. 7 requires a step of inserting the member 22 in order to engage the retaining means of the blood collecting tube with the retaining means of the holder. However, the blood collecting tube 4 can be very easily released from the holder 2 simply by withdrawing the member 22.

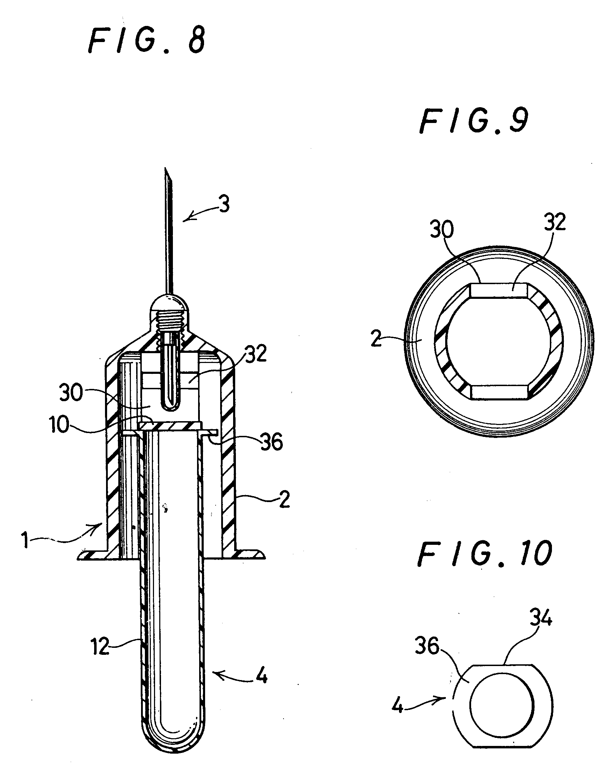

Next, the blood collector according to a yet further embodiment of the present invention is described with reference to FIG. 8. Like parts are designated by like numerals as in FIG. 1.

In the blood collector 1 of this embodiment, the holder 2 is formed by a tubular member having a pair of opposed flat surface portions 30. Each flat surface portion 30 is provided with a slot 32 which communicates the interior and the exterior of the holder 2 and forms the retaining means of the holder 2. The flat surface portion 30 extends up to the rear opening of the holder. FIG. 9 is a cross section of the holder 2 taken along the slots 32. FIG. 10 is a cross section of the blood collecting tube taken along ribs 36. The blood collecting tube 4 includes ribs 36 and straight portions 34 which are cut out so as to match with the shape of the flat surface portions of the holder 2. The ribs 36 form the retaining means of the blood collecting tube 4 and engage the slots 32 of the holder 2. The ribs 36 are engaged with the slots 32 by inserting the blood collecting tube 4 into the holder 2, and rotating the blood collecting tube when the ribs 36 reach the position of the slots 32.

It is also contemplated to use a single rib 36 as well as a single flat surface portion 30. Since the flat surface portion is only required to form a rear end of the slot 32 which is to engage the rib 36, it suffices that the flat surface be present immediately behind the slot 32 (immediately behind the slot on the rear side of the holder). Therefore, the flat surface portion need not extend to the rear end of the holder and may merge into a circular portion contiguous to the remaining portions.

The slots 32 must be longer than the ribs 36. However, too long slots will reduce the strength of the holder. Thus the length of the slots ranges from 2/10 to 7/10, preferably from 4/10 to 6/10 of the entire circumference of the holder. A plurality of slots are preferably formed. Most preferably two slots are formed in an opposed relationship. In this case, the length of each slot ranges from 1/10 to 3.5/10, preferably from 2/10 to 3/10 of the entire circumference of the holder.

Examples of the blood collector of the present invention are presented below.

### Example 1

A tubular member of a shape as shown in FIG. 1 having an interior volume of 10 cc was injection molded from polyethylene terephthalate to form the blood collecting tube. The tubular member at its open end was formed with an annular rib extending outwardly and having a flat rear surface. The annular rib had a thickness of 0.8 mm and its length extending from the outside surface of the tubular member was 1.2 mm. The outside diameter of the tubular member including the annular rib was 16.8 mm. The closure member for the tubular member was formed by using a vulcanized natural rubber sheet (sealing member) of 1.3 mm thick as an upper layer and laminating a polyethylene terephthalate film (of 45 µm thick) having aluminum vapor deposited thereon to the lower surface of the rubber sheet with an epoxy adhesive. The closure member was heat fused to the open end of the tubular member under a certain reduced pressure to form a reduced pressure blood collecting tube.

As the holder for the blood collecting tube, a cylindrical holder as shown in FIG. 1 was formed from polypropylene. The holder at its front end was formed with a threaded bore for thread engagement with a hub of a puncture needle. The holder was formed with an annular groove having a depth of 0.5 mm, a width of 1.0 mm, and a flat rear surface at a position spaced 15.0 mm rearwardly from the front end inside surface. The holder had an inside diameter of 16.9 mm.

To the front end of the holder was mounted a puncture needle in which a hollow needle segment or rear portion of the puncture needle was enclosed by a cylindrical rubber cap having a closed rear end (inside diameter 0.8 mm, wall thickness 0.4 mm).

In this way, the blood collector of the present invention was fabricated.

With the holder and the blood collecting tube of the blood collector held by left and right hands, respectively, the blood collecting tube was inserted into the holder, and then the blood collecting needle segment of the puncture needle was thrusted into a blood vessel. When the blood collecting tube was pushed in until the hollow needle segment pierced throughout the closure member of the blood collecting tube, it was observed that blood entered the blood collecting tube through the puncture needle. When the blood collecting tube was further pushed in, an upper portion of the rib on the blood collecting tube entered an upper portion of the groove in the holder whereby the blood collecting tube was slightly slanted. At this point, the rubber cap was held folded. The blood collecting tube was not moved back even after losing the grip. When the blood collecting tube was gripped at its rear end and manipulated so as to be parallel with the axis of the holder, the blood collecting tube was spontaneously moved back and readily removed.

### Example 2

As the holder for the blood collecting tube, a cylindrical holder as shown in FIG. 3 was formed from polypropylene. The holder at its front end was formed with a threaded bore for thread engagement with the hub of the puncture needle. The holder was formed with two slots having an axial width of 1.2 mm and a length transverse to the axis (arcuate distance) of 15.0 mm, at opposed positions spaced 15.0 mm rearwardly from the front end inside surface. The holder had an inside diameter of 16.9 mm. The blood collecting tube was formed as shown in FIG. 4b which was provided with two ribs each having a thickness of 0.8 mm and an arcuate distance of 10.0 mm at symmetrical positions. The blood collector of the present invention was completed using the same puncture needle as in Example 1.

The blood collecting tube was inserted into the holder of the blood collector in the same manner as in Example 1. When the blood collecting tube was pushed in, an upper portion of the ribs of the blood collecting tube entered the slots at the upper portion of the holder whereby the blood collecting tube was slightly slanted. The blood collecting tube was not moved back even after losing the grip. When the blood collecting tube was gripped at its rear end and manipulated so as to be parallel with the axis of the holder, the blood collecting tube was spontaneously moved back and readily removed.

### Example 3

As the holder for the blood collecting tube, a cylindrical holder as shown in FIG. 5 was formed from polypropylene. The holder at its front end was formed with a threaded bore for thread engagement with the hub of the puncture needle. The holder was formed with an annular rim of a shape having a flat front surface and a moderately slant rear surface at a position spaced 15.0 mm rearwardly from the front end inside surface. The annular rim was 0.5 mm high. The holder had an inside diameter of 16.9 mm at the annular rim. The blood collector of the present invention was completed using the same blood collecting tube and puncture needle as in Example 1.

The blood collecting tube was inserted into the holder of the blood collector in the same manner as in Example 1. When the blood collecting tube was pushed in until an upper portion of the rib of the blood collecting tube passed beyond an upper portion of the annular rim of the holder, the blood collecting tube was slightly slanted. The blood collecting tube was not moved back even after losing the grip. When the blood collecting tube was gripped at its rear end and manipulated so as to be parallel with the axis of the holder, the blood collecting tube was spontaneously moved back and readily removed.

Blood collection using the blood collector of the present invention is described with reference to the embodiment shown in FIG. 1.

First, the blood collecting needle segment 7 of the puncture needle 3 mounted to the holder 2 was thrusted into a blood vessel. Then the blood collecting tube 4 having a reduced pressure interior was inserted into the holder 2 until the free end of the hollow needle segment 8 of the puncture needle 3 pierced throughout the resilient sheath 9 and the closure member 10 of the blood collecting tube 4 and reached the interior of the blood collecting tube 4. Then blood spontaneously entered the blood collecting tube 4. At this point, the resilient sheath 9 was kept folded like bellows between the closure member 10 and the hub 11. When the blood collecting tube 4 was further pushed toward the front end, an upper portion of the retaining means 6 of the blood collecting tube 4 engaged the retaining means 5 of the holder 2 and the blood collecting tube 4 was slightly slanted. If the friction between the closure member 10 of the blood collecting tube 4 and the hollow needle segment 8 prevents the retaining means of the blood collecting tube from being spontaneously engaged with the retaining means of the holder, the blood collecting tube 4 may be slightly slanted to accomplish engagement therebetween. After blood was collected in an amount depending on the reduced pressure in the blood collecting tube 4, the blood collecting tube 4 was gripped at its rear end and manipulated so as to be parallel with the axis of the holder 2. Then the blood collecting tube was spontaneously moved back by the reaction of the folded resilient sheath 9 so that the tube was readily disengaged. The blood collecting tube 4 was completely removed from the holder 2, completing blood collection.

Since the blood collecting tube of the blood collector according to the invention can be inserted into the holder without substantial contact with the holder, the blood collecting tube can be readily inserted into the holder. Since the retaining means of the blood collecting tube engages the retaining means of the holder after insertion, the blood collecting tube is prevented from being moved away by the reaction of the resilient sheath. The blood collecting tube can be readily removed simply by releasing the engagement between the retaining means of the blood collecting tube and the retaining means of the holder.

## Claims

1. A blood collector (1) comprising
- a blood collecting tube (4) having an open end which is closed with a closure member (10);
- a puncture needle (3) including a needle tube having a blood collecting needle segment (7) extending in one direction and a hollow needle segment (8) extending in another direction, and a resilient sheath (9) enclosing the hollow needle segment;
- a holder (2) for holding the blood collecting tube, in the form of a hollow member having an open rear end, the blood collecting tube (4) being removably inserted into the holder (2) through the rear end, the holder (2) being designed such that the blood collecting tube (4) may be inserted into the holder without bringing the outside surface of the tube into substantial contact with the inside surface of the holder, and the puncture needle (3) being mounted at the front end of the hollow member such that the blood collecting needle segment of the puncture needle extends beyond the front end of the hollow member and the hollow needle segment extends through the hollow member,
- wherein the holder (2) and the blood collecting tube (4) are provided with a pair of retaining means (5, 6) for engagement with each other, when the blood collecting tube (4) is inserted into the holder (2),
**characterised** in that
- the inner diameter of the holder (2) and the outer diameter of the blood collecting tube (4) are provided such that there is a clearance between the holder (2) and the blood collecting tube (4) inserted therein to allow the blood collecting tube (4) to be slanted and/or rotated within the holder (2) to thereby achieve engagement therebetween, which engagement provides a retaining force greater than the reaction of the resilient sheath (9) which is axially compressed by the closure member (10) of the blood collecting tube (4) and ensuring safe engagement of the holder (2) and the tube (4).

2. The blood collector of claim 1 wherein the blood collecting tube (4) includes a tubular member having one open end and a closure member (10) closing the opening, the interior of the blood collecting tube (4) being under reduced pressure.

3. The blood collector of claim 2 wherein the retaining means (6) of the blood collecting tube (4) is provided on the tubular member (4).

4. The blood collector of any of claims 1 to 3 wherein the retaining means (6) of the blood collecting tube (4) is a rib outwardly extending from the outside surface of the blood collecting tube.

5. The blood collector of any of claims 1 to 4 wherein the closure member (10) of the blood collecting tube (4) is a film-shaped member having gas barrier properties and sealing ability to seal a puncture opening after the once punctured hollow needle segment (8) is withdrawn.

6. The blood collector of any of claims 1 to 5 wherein the retaining means (5) of the holder is a recess in the inside surface of the holder.

7. The blood collector of any of claims 1 to 5 wherein the retaining means (5) of the holder (2) is a protrusion inwardly extending from the inside surface of the holder.

8. The blood collector of any of claims 1 to 5 wherein the retaining means (5) of the holder (2) is a slot in the holder.

9. The blood collector of any of claims 1 to 5 wherein the retaining means (20) of the holder (2) is a slot into which a plate or rod-shaped member (22) is inserted, and the retaining means (6) of the tube (4) is a protrusion or rib outwardly extending from its outside surface.

## Patentansprüche

1. Blutsammeleinrichtung (1), umfassend
- eine Blutsammelröhre (4), die ein offenes Ende aufweist, das mittels eines Verschlußelements (10) verschlossen ist;
- eine Einstechnadel (3), die eine Nadelröhre umfaßt, die einen sich in eine Richtung erstreckenden Blutsammelnadelabschnitt (7) und einen sich in die andere Richtung erstreckenden Hohlnadelabschnitt (8) und eine elastische Hülle (9) aufweist, die den Hohlnadelabschnitt umgibt;
- eine Haltevorrichtung (2) zum Halten der Blutsammelröhre, in der Form eines Hohlelements, das ein offenes hinteres Ende aufweist, wobei die Blutsammelröhre (4) durch das hintere Ende entfernbar in die Haltevorrichtung (2) eingeführt wird, wobei die Haltevorrichtung (2) derart konstruiert ist, daß die Blutsammelröhre (4) in die Haltevorrichtung eingeführt werden kann, ohne daß die Außenfläche der Röhre in wesentlichen Kontakt mit der Innenfläche der Haltevorrichtung gebracht wird, und wobei die Einstechnadel (3) am vorderen Ende des Hohlelements derart angebracht ist, daß sich der Blutsammelnadelabschnitt der Einstechnadel über das vordere Ende des Hohlelements hinaus erstreckt und sich der Hohlnadelabschnitt durch das Hohlelement erstreckt,
- wobei die Haltevorrichtung (2) und die Blutsammelröhre (4) mit einem Paar Haltemittel (5, 6) für einen Eingriff miteinander versehen sind, wenn die Blutsammelröhre (4) in die Haltevorrichtung (2) eingeführt wird,
dadurch **gekennzeichnet,** daß
- der Innendurchmesser der Haltevorrichtung (2) und der Außendurchmesser der Blutsammelröhre (4) derart vorgesehen sind, daß ein Zwischenraum zwischen der Haltevorrichtung (2) und der darin eingeführten Blutsammelröhre (4) vorhanden ist, um es zu ermöglichen, daß die Blutsammelröhre (4) innerhalb der Haltevorrichtung (2) schräg gestellt und/oder gedreht wird, um dadurch zwischen ihnen einen Eingriff zu erreichen, welcher Eingriff eine Haltekraft bereitstellt, die größer als die Gegenwirkung der elastischen Hülle (9) ist, die axial durch das Verschlußelement (10) der Blutsammelröhre (4) zusammengedrückt wird und einen sicheren Eingriff der Haltevorrichtung (2) und der Röhre (4) sicherstellt.

2. Blutsammeleinrichtung nach Anspruch 1, bei der die Blutsammelröhre (4) ein röhrenförmiges Element umfaßt, das ein offenes Ende und ein Verschlußelement (10) aufweist, das die Öffnung schließt, wobei das Innere der Blutsammelröhre (4) unter vermindertem Druck steht.

3. Blutsammeleinrichtung nach Anspruch 2, bei der das Haltemittel (6) der Blutsammelröhre (4) am röhrenförmigen Element (4) vorgesehen ist.

4. Blutsammeleinrichtung nach einem der Ansprüche 1 bis 3, bei der das Haltemittel (6) der Blutsammelröhre (4) eine sich von der Außenfläche der Blutsammelröhre nach außen erstreckende Rippe ist.

5. Blutsammeleinrichtung nach einem der Ansprüche 1 bis 4, bei der das Verschlußelement (10) der Blutsammelröhre (4) ein folienförmiges Element ist, das Gasbarriereneigenschaften und Abdichtungsvermögen aufweist, um eine Einstechöffnung abzudichten, nachdem der einmal eingestochene Hohlnadelabschnitt (8) herausgezogen ist.

6. Blutsammeleinrichtung nach einem der Ansprüche 1 bis 5, bei der das Haltemittel (5) der Haltevorrichtung eine Vertiefung in der Innenfläche der Haltevorrichtung ist.

7. Blutsammeleinrichtung nach einem der Ansprüche 1 bis 5, bei der das Haltemittel (5) der Haltevorrichtung (2) ein sich von der Innenfläche der Haltevorrichtung nach innen erstreckender Vorsprung ist.

8. Blutsammeleinrichtung nach einem der Ansprüche 1 bis 5, bei der das Haltemittel (5) der Haltevorrichtung (2) ein Schlitz in der Haltevorrichtung ist.

9. Blutsammeleinrichtung nach einem der Ansprüche 1 bis 5, bei der das Haltemittel (20) der Haltevorrichtung (2) ein Schlitz ist, in den ein platten- oder stangenförmiges Element (22) eingeführt wird, und das Haltemittel (6) der Röhre (4) ein sich von ihrer Außenfläche nach außen erstreckender Vorsprung oder eine Rippe ist.

## Revendications

1. Dispositif (1) de prélèvement sanguin qui comprend :
- un tube (4) de prélèvement de sang ayant une extrémité ouverte qui est fermée par un élément de fermeture (10),
- une aiguille de ponction (3) comprenant une aiguille tubulaire avec un segment d'aiguille (7) de prélèvement de sang s'étendant dans une direction et un segment d'aiguille creuse (8) s'étendant dans une autre direction, et un fourreau élastique (9) enfermant le segment d'aiguille creuse,
- un support (2) pour tenir le tube de prélèvement de sang, en forme d'élément creux ayant une extrémité arrière ouverte, le tube (4) de prélèvement de sang étant introduit de façon amovible dans le support (2) par l'extrémité arrière, le support (2) étant conçu pour que le tube (4) de prélèvement de sang puisse être introduit dans le support sans amener la surface extérieure du tube en contact substantiel avec la surface intérieure du support, et l'aiguille de ponction (3) étant montée à l'extrémité avant de l'élément creux de sorte que le segment d'aiguille de prélèvement de sang de l'aiguille de ponction s'étende au-delà de l'extrémité avant de l'élément creux et que le segment d'aiguille creuse traverse l'élément creux,
dans lequel le support (2) et le tube (4) de prélèvement de sang sont dotés d'une paire de moyens de retenue (5, 6) destinés à s'accrocher l'un avec l'autre quand le tube (4) de prélèvement de sang est introduit dans le support (2),
caractérisé en ce que le diamètre intérieur du support (2) et le diamètre extérieur du tube (4) de prélèvement de sang sont tels qu'il existe un jeu entre le support (2) et le tube (4) de prélèvement de sang introduit dans celui-ci pour permettre au tube (4) de prélèvement de sang d'être incliné et/ou tourné à l'intérieur du support (2) pour réaliser ainsi l'accrochage entre eux, lequel accrochage donne une force de retenue plus grande que la réaction du fourreau élastique (9) qui est comprimé axialement par l'élément de fermeture (10) du tube (4) de prélèvement de sang et garantit un accrochage sûr du support (2) et du tube (4).

2. Dispositif de prélèvement sanguin selon la revendication 1, dans lequel le tube (4) de prélèvement de sang comprend un élément tubulaire ayant une extrémité ouverte et un élément de fermeture (10) qui ferme l'ouverture, l'intérieur du tube (4) de prélèvement de sang étant sous une pression réduite.

3. Dispositif de prélèvement sanguin selon la revendication 2, dans lequel le moyen de retenue (6) du tube (4) de prélèvement de sang est placé sur l'élément tubulaire (4).

4. Dispositif de prélèvement sanguin selon l'une quelconque des revendications 1 à 3, dans lequel le moyen de retenue (6) du tube (4) de prélèvement de sang est une nervure s'étendant vers l'extérieur depuis la surface extérieure du tube de prélèvement de sang.

5. Dispositif de prélèvement sanguin selon l'une quelconque des revendications 1 à 4, dans lequel l'élément de fermeture (10) du tube (4) de prélèvement de sang est un élément de type pelliculaire ayant des propriétés de barrière aux gaz et une capacité d'étanchéité pour fermer un orifice de perforation une fois que l'on a retiré le segment d'aiguille creuse (8) qui a été enfoncé.

6. Dispositif de prélèvement sanguin selon l'une quelconque des revendications 1 à 5, dans lequel le moyen de retenue (5) du support est un évidement dans la surface intérieure du support.

7. Dispositif de prélèvement sanguin selon l'une quelconque des revendications 1 à 5, dans lequel le moyen de retenue (5) du support (2) est une saillie vers l'intérieur qui part de la surface intérieure du support.

8. Dispositif de prélèvement sanguin selon l'une quelconque des revendications 1 à 5, dans lequel le moyen de retenue (5) du support (2) est une fente dans le support.

9. Dispositif de prélèvement sanguin selon l'une quelconque des revendications 1 à 5, dans lequel le moyen de retenue (20) du support (2) est une fente dans laquelle est introduit un élément (22) en forme de plaque ou de barre et le moyen de retenue (6) du tube (4) est une saillie ou nervure s'étendant vers l'extérieur depuis sa surface extérieure.
